# EUROPEAN PATENT APPLICATION

(11) **EP 1 655 049 A1**
(43) Date of publication of application: **10.05.2006**
(21) Application number: 05110029.5
(22) Date of filing: 26.10.2005
(51) Int. Cl.: A61M 11/02

(54) **Aerosol therapy apparatus with controllable flow**

(30) Priority: 03.11.2004 IT MI20042104
(71) Applicant: MED 2000 S.r.l., 25080 Padenghe Sul Garda BS (IT)
(72) Inventor: Fraccaroli, Nicola, 25080, PEADENGHE SUL GARDA (BS) (IT)
(74) Representative: Giambrocono, Alfonso

(57) **Abstract**

The aerosol therapy apparatus (A, B) is of the type in which the air flow reaching the nebulizer chamber (13) is generated by a piston compressor (11). The apparatus is provided with a flow regulator device (14) and a flow measurement device (16). This enables the working flow to be controlled, to achieve the appropriate value for obtaining the particulate particle size distribution declared by the manufacturer.

## Description

The present invention relates to an aerosol therapy apparatus of the type in which the air flow reaching the nebulizer chamber is generated by a piston compressor.

If intended for "professional" use, current apparatuses of this type are provided, in contrast to those for "domestic use" (which do not have them), with a pressure gauge which enables the operating pressure of the air flow to be monitored.

According to the new Medical Devices Directive 93/42/CEE harmonized standard EN13544-1, instrumentation in the form of said pressure gauge is totally inadequate and actually gives false information. In this respect, the performance of a nebulizer chamber (which is the main element of the aerosol therapy apparatus and constitutes its load) is evaluated in the official literature, as requested by the competent controlling bodies (in particular the E.R.S.), on the basis of tests relative to working flows.

It should however be noted that different nebulizer chambers constitute a different load if using the same compressor. The load variation between different nebulizer chambers is due mainly to differences in the diameter of the nebulizer chamber exit hole, which in commercially available nebulizer chambers is between 0.4 and 0.6 mm.

As the working point of an aerosol therapy apparatus is given by the intersection of the compressor load curve and the nebulizer chamber load curve, a different nebulizer chamber displaces the working point along the compressor load curve, altering both the flow and the operating pressure of the apparatus. As there is no biunique correspondence between flow and pressure, it follows that even though apparatuses intended for professional use are provided with a pressure gauge for pressure control, this instrument does not enable the user to obtain a reading of the apparatus performance expressed on the basis of the working flow advised by the manufacturer, or of the performance useful to the user.
Experiments carried out by the inventors of the aerosol therapy apparatus according to the present invention have shown that the performance of an apparatus of this type depends in the main not only on the type of nebulizer chamber used, but also (for the same nebulizer chamber) on the working flow.

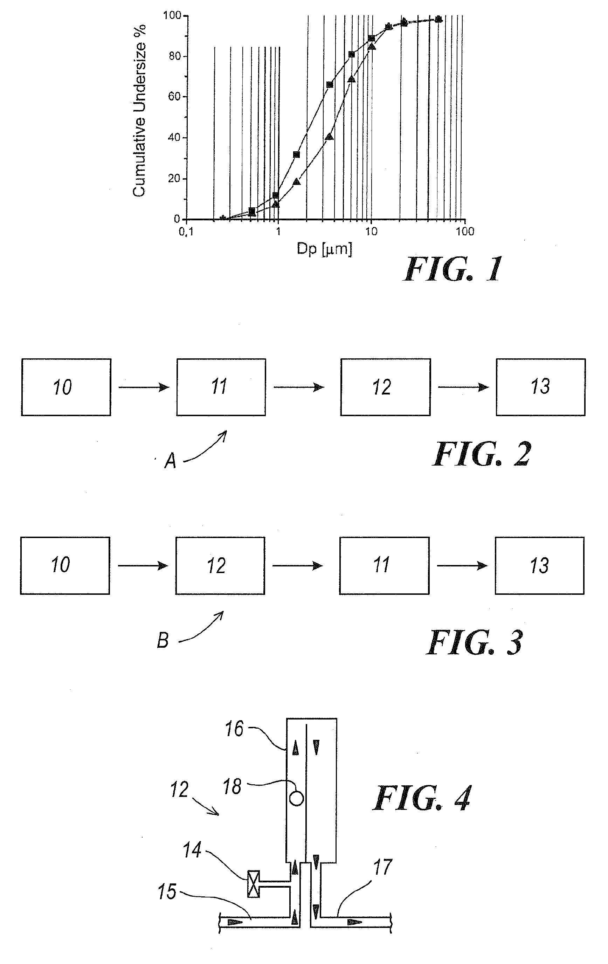

Tests carried out at the University of Bradford, Great Britain, on the AndyFlow® nebulizer chamber produced by the proprietor of the present patent application, using the Andersen cascade impactor (A.C.I.), in conformity with the aforesaid standard, have shown a strong performance variation with varying flow. For the aforesaid nebulizer chamber the accompanying Figure 1 shows a graph in which the horizontal axis indicates the particle diameter (indicated by Dp) on a logarithmic scale, and the vertical axis indicates the cumulative undersize, i.e. the percentage of particles less than a determined diameter (which is one of the parameters considered by the standard). This graph shows the performance of a determined nebulizer chamber and the dependence of performance on the working flow. In the specific case of the aforesaid nebulizer chamber, the points indicated on the graph by a triangle were obtained using a flow of 5 litres/minute. The points indicated by a square were obtained by repeating the test with a flow of 8 litres/minute. By interpolating the relative points the two curves of the graph are obtained, which as can be seen are quite different, indicative of a significant performance variation as the flow varies.

As the aforesaid standard requires the manufacturer of a aerosol therapy apparatus of professional type to state the size distribution of the particles emitted by the apparatus (this distribution being related to deposition of the active principle), it becomes important to always obtain the best possible performance achievable by a determined apparatus for a determined nebulizer chamber. As is well known, this performance depends on the following parameters: MMAD, GSD, OUTPUT, OUTPUT RATE. For the meaning of these latter, which are in any event clear to the expert of the art, reference should be made to the aforesaid standard.

The object of the present invention is to provide an aerosol therapy apparatus which can always be set to provide maximum performance, even when replacing the nebulizer chamber with another of different type (i.e. even if the load is changed).

This object is attained by the aerosol therapy apparatus according to the present invention, characterised by being provided with a flow regulator device and a flow measurement device. The working flow can hence be controlled to the value appropriate for obtaining the particulate particle size distribution declared by the manufacturer (as required by said standard), to hence achieve the required active principle deposition.
Conveniently, the flow measurement device used is a compensated pressure flowmeter which can be calibrated on the apparatus itself, the flow regulator device used being a needle valve.

The aerosol therapy apparatus of the present invention ensures a constant working flow adjustable to the required value on the basis of the load (i.e. the nebulizer chamber used in the apparatus).

The invention will be more apparent from the ensuing description of two embodiments thereof. This description refers to the accompanying Figures 2 to 4, in which:
Figure 2 shows the operating scheme of a first aerosol therapy apparatus according to the invention;
Figure 3 shows the operating scheme of a second aerosol therapy apparatus according to the invention;
Figure 4 shows very schematically an embodiment of a combination comprising both the flow measurement device and the flow regulator device, this combination being usable in both the apparatuses of Figures 2 and 3.

In the aerosol therapy apparatus shown schematically in Figure 2 and indicated overall by A, the path of the air flow between the various apparatus components, generated by a conventional piston compressor 11, is shown by arrows and originates at a conventional air filter 10 positioned at the intake port of the compressor 11. The flow then leaves from the delivery port of the compressor 11, this port communicating with a flow regulator device, downstream of which there is a flow measurement device (the combination of these two devices being indicated in Figure 2 by 12). The flow finally reaches a conventional nebulizer chamber 13 also forming part of the apparatus A.

The apparatus shown schematically in Figure 3 and indicated overall by B differs from that (A) of Figure 2 in that the compressor 11 is positioned not upstream but downstream of the combined flow regulator device and flow measurement device 12.

As stated, the combination 12 comprises the flow regulator device and the flow measurement device. In both the apparatuses A and B it conveniently consists (Figure 4) of a needle valve 14, downstream of which a flowmeter 16 is provided. The valve 14 enables the air flow to be regulated, this flow originating directly from the filter 10 in the case of the apparatus A (Figure 2) and from the filter 10 via the compressor 11 in the case of the apparatus B (Figure 3), the flow reaching the valve 14 via a channel 15 (Figure 4), to then pass through the flowmeter 16. This latter is of the compensated pressure type and comprises a steel ball 18 which, under the action of the air flow, is urged upwards until it reaches an equilibrium position in which its weight compensates the upward thrust exerted on it by the air flow. The position reached by the ball 18 is hence indicative of the value of the apparatus working flow, a value which can be read by providing on the casing of the flowmeter 16 - formed of a transparent material so that the ball 18 is visible from the outside - a suitable scale (for example in litres/minute) to enable the relative flow value to be read.

The compressed air flow leaving the flowmeter 16 passes through a channel 17 leading directly to a nebulizer chamber 13 (constituting the load) in the case of the apparatus A (Figure 2) whereas in the case of the apparatus B the channel 17 leads to the intake port of the compressor 11, the delivery port of which is directly connected to the nebulizer chamber 13.

By acting on the needle valve 14, the flow reaching the nebulizer chamber 13 can be varied, to enable it to assume the required value (indicated by the position of the ball 18), this value being previously determined such as to obtain the required performance based on the load. It is important to note that in the case of the apparatus B (Figure 3) there is the important advantage that adjusting the flow by means of the valve 14 does not alter the load at the compressor exit (as instead happens in the case of the apparatus A), so that the power absorbed by the compressor does not vary as a result of adjusting the flow.

By virtue of the present invention, an aerosol therapy apparatus can be provided with different nebulizer chambers intended for different types of therapy, provided that for each nebulizer chamber the appropriate flow value (corresponding to the maximum performance for that nebulizer chamber) is indicated. The flow value corresponding to the maximum performance for a determined nebulizer chamber can be set directly by the user by simply operating the needle valve 14.
It should also be noted that as determined dimensional tolerances are allowable when manufacturing a nebulizer chamber, even small variations in the diameter of the nebulizer chamber exit hole (due to the determined dimensional tolerances) result in a load variation which can lead to a large performance reduction. In this case, as the flow can be varied by means of the needle valve 14, the user is able to directly return the flow to the prescribed optimal value.

Another advantageous result is that in manufacturing the nebulizer chamber, wider dimensional tolerances can be set (with a reduction in costs), as the flow can in any event be adjusted to obtain the required performance.

## Claims

1. An aerosol therapy apparatus (A, B) of the type in which the air flow reaching the nebulizer chamber (13) is generated by a piston compressor (11), **characterised by** being provided with a flow regulator device (14) and a flow measurement device (16).

2. An aerosol therapy apparatus (A) as claimed in claim 1, wherein the regulator device (14) and the measurement device (16) are positioned downstream of the compressor (11).

3. An aerosol therapy apparatus (B) as claimed in claim 1, wherein the regulator device (14) and the measurement device (16) are positioned upstream of the compressor (11).

4. An aerosol therapy apparatus (A, B) as claimed in claim 1, wherein a compensated pressure flowmeter (16) is used as the flow measurement device.

5. An aerosol therapy apparatus (A, B) as claimed in claim 1, wherein a needle valve (14) is used as the flow regulator device.
